# EUROPEAN PATENT APPLICATION

(11) **EP 1 090 544 A1**
(43) Date of publication of application: **11.04.2001**
(21) Application number: 99919026.7
(22) Date of filing: 06.05.1999
(51) Int. Cl.: A01G 7/00, A01H 3/00, A01H 5/10

(54) **A PROCESS FOR PRODUCING CORN GRAIN BY USING THREE TYPES OF GENETIC EFFECT**

(30) Priority: 12.05.1998 CN 98101902
(71) Applicant: China Agricultural University, Haidian District, Beijing 100094 (CN)
(72) Inventor: SONG, Tongming, Haidian District, Beijing 100094 (CN)
(74) Representative: UEXKÜLL & STOLBERG
(86) International application number: CN9900065
(87) International publication number: WO9957960

(57) **Abstract**

A process for producing high yield corn with enhanced grain quality traits is disclosed, whereby the seeds of the interlinear hybrid corn with cytoplasm male sterility and the seeds of the normal pollinator hybrid corn having significant economic traits such as high oil or high protein are mixedly planted in a ratio of 4-9:1. In this process, the effect of cytoplasm male sterility on increasement of yield of the grain is fully used, the effect of mere planting of the interlinear hybrid on the degeneration of the grain is successfully avoided and the output-increasing effect gained from heterosis caused by re-crossing interlinear hybrid is utilized. And, characters of father hybrid corn, including high oil and other economic characters controlled by major dominant gene or semi-dominant gene, have been expressed by grains of mother interlinear hybrid corn through metaxenia.

## Description

### Field of the invention

The present invention is related to a method of corn production, specifically a method of enhanced quality, high yield corn production that utilizes three genetic effects.

### Background of the invention

Among current corn production methods, there have been double-cross hybrid (inbred A X inbred B) X (inbred C X inbred D), triple-cross hybrid (inbred A X inbred B) X inbred C, and multiple-cross hybrid (produced by hybridization of multiple inbred or hybrid seeds). But since the 60s, single-cross hybrid (inbred A X inbred B) has been the predominant production method of regular corn as well as specialty corn including high-lysine corn, sweet corn, high-branching-starch corn, and glutinous corn. In the production of high-oil corn, although the single-cross hybrid is also utilized, it is often produced from the hybridization between a regular inbred corn (low-oil) and a high-oil inbred corn. Recently in the U. S. corn seeds containing 2-7.5% oil with 55% oleic acid have been produced by hybridization between a high-oleic acid mutant corn female parent after mechanical detasselling, or chemical sterilization or being made genetic male sterile, and a male parent containing high-oil, or high-oleic acid, or both high-oil and high-oleic acid. The oil produced from these corn grains has stable anti-oxidation property for food use and animal feed. However, the hybrid vigor from the re-hybridization of the single-cross hybrid was not mentioned. Cytoplasmic male sterility was treated as a method of hybridization like mechanical detasselling or chemical sterilization, its potential for yield increase was not mentioned. Both of the male parent and the female parent were high-oleic acid for maintaining the anti-oxidation stability of the corn oil, the use of the pollen-determinant effect of many quality traits on corn quality improvement, such as protein content and oil content, was not elucidated. The commonly used single-cross hybrid planting model in current corn production only utilizes the hybrid vigor of the F1 generation derived from two inbred parents; it does not prevent yield deterioration caused by the selfing of the F1 plants, nor it utilizes the hybrid vigor of the seed resulted from the re-hybridization of two single-cross hybrids. The yield-enhancing effect of corn cytoplasmic male sterility was not utilized; furthermore, the great improvement of corn quality due to the pollen-determinant effect is not utilized.

### Invention description

The objective of the present invention is to provide a method of producing enhanced quality and high yielding corn. In this method while the hybrid vigor of the F1 generation plant of the single-cross hybrid is sustained, the seed heterosis generated by the re-hybridization of the F1 generation (e.g. single-cross hybrid A X single-cross hybrid B) is utilized; the yield-enhancing effect of corn cytoplasmic male sterility is also utilized; meanwhile the pollen-determinant effect on elevating protein, oil (lipid) content and quality improvement is also utilized. Comparing to sole planting of single-cross hybrid, this method leads corn production to higher yield, higher oil content, greatly improved grain quality, and therefore the realization of the dual benefits of enhanced quality and high yield.

The present invention is about a method of corn production, which is:

To produce high quality corn resulted from the pollen-determinant effect on the pollen receiving kernel by mix-planting the cytoplasmic male sterile single-cross hybrid with the male fertile and high grain quality pollen donor corn hybrid seeds in a ratio of 4-9:1, or by planting in separate rows in the same ratio instead of mix-planting. Pollen-determinant effect described in the present invention should include the pollen-determinant effects of qualitative traits and the pollen-determinant effects of quantitative traits.

The corn production method described in the present invention utilizes the yield-increasing effect of cytoplasmic male sterility, the yield-increasing effect of the heterosis derived from the re-hybridization of the single-cross hybrids, and the quality-improving function of pollen-determinant effect. The cytoplasmic male sterility described in the present invention includes T-type cytoplasmic male sterility, C-type cytoplasmic male sterility, S-type cytoplasmic male sterility, and other types such as Y-type cytoplasmic male sterility.

The re-hybridization of single-cross hybrid described in the present invention includes the hybridization between any type of cytoplasmic male sterile single-cross hybrid and another genetically distant hybrid which provides pollen to the male sterile hybrid and thus is called the pollen donor. The hybridization of the pollen donor and the male sterile single-cross hybrid not only causes heterosis on the pollen-receiving kernel, but also brings in important qualitative or quantitative traits that can cause pollen-determinant effect.

The quantitative traits having pollen-determinant effect described in the present invention refer to large kernel size, high oil (fat) content, high protein content and semi-dominant or cumulative grain quality traits such as high-lysine content, high-methionine content and etc.

The large kernel size described in the present invention refers to kernels of greater than 350 mg. The high-oil content refers to seeds containing oil at greater than 6%. The high-protein content refers to seeds containing protein at greater than 12%. The high lysine content refers to seeds containing lysine at greater than 0.26%. The high methionine content refers to seeds containing methionine at greater than 0.20%.

The quality traits having pollen-determinant effect described in the present invention include the major dominant gene controlled corn kernel color aleurone layer traits and the starchy endosperm layer traits. The major dominant genes controlling the color aleurone layer traits are A₁, A₂, C₁, C₂, Bz₁, Bz₂, R₁ and Pr. The major dominant genes controlling the starchy endosperm layer traits are Y, Wx, O₂, Ae, Se, Sh, Su, Bt, and etc. The major recessive genes controlling the starchy endosperm layer traits are y, wx, o₂, ae, se, sh, su, and etc.

The present invention can be practiced in agricultural production as following: The cytoplasmic male sterile single-cross hybrid is mix- or separate-planted with pollen donor corn in a ratio of 4-9:1 so that corn grains are produced.

The pollen donor corn hybrid described in the present invention refers to single-cross hybrid, double-cross hybrid, triple-cross hybrid or multiple-cross hybrid.

The seeds produced by hybridization of male sterile single-cross hybrid and a pollen donor or by out-crossing among the pollen donor hybrid plants are all within the scope of the present invention. These seeds can only be used for food, animal feed or industrial materials and can not be planted.

One aspect of the present invention is to utilize cytoplasmic male sterility in corn production so that the single-cross hybrid used in the current production can be cytoplasmic-male-sterilized. The sterilized single-cross hybrid has dual functions in the present invention: First, because this sterilized single-cross hybrid has lost its ability to produce pollen, it can reduce apical dominance and promote female ear development; meanwhile the nutrients otherwise needed for pollen production can be transferred to seed. Therefore, comparing to the same type regular single-cross hybrid, the sterile hybrid has higher yield potential. Secondly, the sterile single-cross hybrid is incapable of selfing or outcrossing with a close relative, therefore, the kernel deterioration in F2 generation caused by the out-crossing among close relatives of regular single-cross hybrid is prevented. This deterioration not only can result in lower kernel uniformity, i.e. reduced kernel weight and number of kernel, but also can affect its disease resistance and many other agronomic traits. Another aspect of the present invention is to plant, mixed or in separate rows, in certain ratio the single-cross hybrid that is capable of shedding pollens as pollen donor and the sterile single-cross hybrid. The pollen shedding time of the pollen donor should be in sync with the silk emerging time of the sterile single-cross hybrid. The pollen donor should be genetically different from or genetically complementary to the sterile single-cross hybrid so that the pollen donor can induce heterosis and increase yield of the sterile single-cross hybrid. The pollen recipient should also have strong economic traits that have pollen-determinant effect such as high-oil content (above 6%), high-protein content (above 12%), or certain cumulative quantitative traits such as large kernel size, high lysine, high methionine, or the major gene controlled dominant qualitative traits such as the color aleurone layer traits and the yellow starchy endosperm layer traits. These traits of the pollen donor can be exhibited in the kernel of the male sterile single-cross hybrid due to the pollen-determinant effect.

The operational procedures of the above-described invention: Before sowing, the seeds of the cytoplasmic male sterile single-cross hybrid and the seeds of the pollen donor hybrid with high-oil or other high quality traits are mixed in certain ratio. The mixed seeds can be sown in the field. Alternatively the two types of seeds can be sown in separate rows. The seeds produced by the sterile plant will be the high yielding, high oil and high quality corn intended by the present invention that account for greater than 80% of the total harvest. The seeds produced by the pollen donor plants account for less than 20% of the total harvest and are produced by out-crossing of the close relatives of the pollen donor. These two types of harvested seeds can be mixed or separated for commercial use.

### Preferred embodiments of the invention

The following is the detailed description of the present invention with examples of practical applications.

### EXAMPLE 1. Production of high yield, high-oil commercial corn.

The current commonly used regular corn hybrid (e.g. 4% oil) is converted into cytoplasmic male sterile. Thus obtained male sterile seeds are mixed prior to sowing in a ratio of 4-8:1 with a specific high oil corn hybrid pollen recipient (14% oil) having the same reproductive cycle time and planted. Or alternatively the seeds are sown in separate rows in the same ratio instead of mix-planting. The yield of corn grains of the male sterile single-cross hybrid plants is 1-8% higher than that of the corresponding regular fertile corn planted alone. The oil content increases by 70-80% to reach oil content of 7-8%. The corn grains of the high oil hybrid pollen recipient plants remain 14% in oil content. No matter harvested separately or mixed, both types of corn grains belong to the high yield and high-oil corn as intended in the present invention.

### EXAMPLE 2. Production of high yield, purple sweet commercial corn.

Corn seeds of cytoplasmic male sterile white sweet or yellow sweet single-cross hybrid are mixed prior to sowing with the corn seeds of a purple sweet hybrid pollen recipient in a ratio of 4-8:1 and planted. Or alternatively the seeds are sown in separate rows in the same ratio instead of mix-planting. The yield of sweet corn from the male sterile plants increases by 4-10% over that of the corresponding regular fertile corn planted alone. Because of the pollen-determinant effect of the purple genes (A₁, A₂, C₁, C₂, Bz₁, Bz₂, Pr), all the corn produced are purple sweet corn. All the purple sweet corn harvested from both the male sterile plants and the pollen donor plants belong to the high yield, purple sweet commercial corn as intended in the present invention.

### EXAMPLE 3. Production of high yield, yellow glutinous commercial corn.

Corn seeds of the cytoplasmic male sterile white glutinous corn hybrid are mixed prior to sowing with the corn seeds of a yellow glutinous corn hybrid pollen donor in a ratio of 4-8:1 and planted. Or alternatively the seeds are sown in separate rows in the same ratio instead of mix-planting. Due to the pollen-determinant effect of the yellow endosperm genes, the corn produced by the male sterile white glutinous plants are all yellow glutinous corn. Because of the yield-enhancing effect of male sterility and the heterosis from re-hybridization of two hybrids, the yield of corn from the male sterile plants increases by 4-10% over that of the corresponding regular hybrids planted alone.

### EXAMPLE 4. Production of high yield, purple glutinous commercial corn.

Corn seeds of the cytoplasmic male sterile white glutinous corn or yellow glutinous corn hybrids are mixed prior to sowing with the corn seeds of a purple glutinous corn hybrid pollen recipient in a ratio of 4-8:1 and planted. Or alternatively the seeds are sown in separate rows in the same ratio instead of mix-planting. The yield of corn from the male sterile plants increases by 1-8% over that of the corresponding regular single-cross hybrid planted alone. Because of the pollen-dominant effect of the purple genes, all the corn produced by the male sterile plants become purple glutinous corn.

### EXAMPLE 5. Production of high yield, high-oil, purple glutinous commercial corn.

Corn seeds of the cytoplasmic male sterile white glutinous corn hybrid (oil content 3-5%) are mixed prior to sowing with corn seeds of high-oil (oil content above 9%) purple glutinous corn hybrid pollen recipient in a ratio of 4-8:1 and planted. Or alternatively the seeds are sown in separate rows in the same ratio instead of mix-planting. The corn produced by the male sterile glutinous corn hybrid plants not only are high oil (oil content above 5%) but also are purple. The yield is no less than that of the corresponding regular fertile single-cross hybrid planted alone or even an increase of 1-5%.

### EXAMPLE 6. Production of high yield, high-lysine commercial corn.

Corn seeds of the cytoplasmic male sterile regular corn hybrid (lysine content 0.2-0.24%) are mixed prior to sowing with corn seeds of the high-lysine (lysine content above 0.4%) corn hybrid pollen recipient that carries dominant or semi-dominant high-lysine genes in a ratio of 4-8:1 and planted. Or alternatively the seeds are sown in separate rows in the same ratio instead of mix-planting. The corn harvested from the male sterile low-lysine hybrid plants is now high-lysine (lysine content above 0.3%). The yield is also 2-10% higher than that of the corresponding regular single-cross hybrid corn planted alone.

### EXAMPLE 7. Production of high yield, high-methionine commercial corn.

Corn seeds of the cytoplasmic male sterile regular corn hybrid (methionine content 0.2%) are mixed prior to sowing with corn seeds of the high-methionine (methionine content above 0.35%) corn pollen recipient that carries dominant or semi-dominant high methionine content genes and modifier genes, in a ratio of 4-8:1 and planted. Or alternatively the seeds are sown in separate rows in the same ratio instead of mix-planting. The corn harvested from the male sterile regular hybrid plants is now high-methionine (methionine content above 0.25%). The yield is also 2-10% higher than that of the corresponding regular fertile corn planted alone.

### EXAMPLE 8. Production of high yield, high-oil, high-lysine commercial corn.

Corn seed of the high-yielding cytoplasmic male sterile single-cross hybrid are mixed prior to sowing with the corn seeds of the high-oil (oil content above 9%) and high-lysine (lysine content above 0.3%) hybrid pollen recipient that canies dominant or semi-dominant high-lysine genes, in a ratio of 4-8:1 and planted. Or alternatively the seeds are sown in separate rows in the same ratio instead of mix-planting. The corn harvested from the male sterile hybrid plants yield 1-5% higher than that of the corresponding fertile corn planted alone; and contain 1.5% more oil and 0.1% more lysine. So these corn become high yield, high oil and high lysine corn.

### EXAMPLE 9. Production of high yield, high-oil, high-methionine commercial corn.

Corn seeds of the high yielding cytoplasmic male sterile single-cross hybrid are mixed prior to sowing with corn seeds of the high-oil (oil content above 9%) and high-methionine (methionine content above 0.35%) hybrid corn pollen recipient that carries dominant or semi-dominant high-methionine genes, in a ratio of 4-8:1 and planted. Or alternatively the seeds are sown in separate rows in the same ratio instead of mix-planting. The corn harvested from the male sterile hybrid plants yield 1-5% higher than that of the corresponding fertile corn planted alone; and contain 1.5% more oil and 0.1% more methionine. So these corn become high yield, high oil and high methionine corn.

### EXAMPLE 10. Production of high yield, high-oil, high-lysine, high-methionine commercial corn.

Corn seeds of the high yielding cytoplasmic male sterile single-cross hybrid are mixed prior to sowing with corn seeds of high-oil (oil content above 9%), high-lysine (lysine content above 0.4%) and high-methionine (methionine content above 0.35%) corn hybrid pollen recipient that carries dominant or semi-dominant high lysine genes and dominant or semi-dominant high-methionine genes, in a ratio of 4-8:1 and planted. Or alternatively the seeds are sown in separate rows in the same ratio instead of mix-planting. The corn harvested from the male sterile hybrid piants yield 1-5% higher than that of the corresponding fertile corn planted alone; and contain 1.5% more oil, 0.1% more lysine and 0.07% more methionine. So these corn become high yield, high oil high lysine and high methionine corn.

### EXAMPLE 11. Production of colored, enhanced sweet and glutinous commercial corn.

Corn seeds of the high yielding cytoplasmic male sterile glutinous corn (wxwxSususese) hybrid carrying the sweet endosperm gene (su), modifier gene (se) and colorless aleurone layer are mixed in a ratio of 4-8:1 prior to sowing with corn seeds of hybrid pollen recipient that carries the color aleurone layer genes (homozygous A₁, A₂, C₁, C₂, Bz₁, Bz₂, Pr) and enhanced sweetness genes (homozygous wx, su, se), in a ratio of 4-8:1 and planted. Or alternatively the seeds are sown in separate rows in the same ratio instead of mix-planting. The corn harvested from the male sterile plants yield 2-10% higher than that of the corresponding fertile corn hybrids planted alone. The aleurone layer of these corn grains appear colored. Because of the segregation of Su and su genes 50% of the corn grains have glutinous corn (wx, Su, Se) phenotype and 50% of the corn grains have enhanced sweetness corn (wx, su, se) phenotype. Thus these corn become the high yield, colored, glutinous and enhanced sweet corn.

### EXAMPLE 12. Production of high yield, large kernel size commercial corn.

Corn seeds of the cytoplasmic male sterile regular hybrid are mixed prior to sowing with corn seeds of a large kernel size (greater than 400 g/1000 kernels) corn hybrid pollen recipient that has similar reproduction cycle time but is genetically distant, and has high compatibility and strong heterosis, in a ratio of 4-8:1 and planted. Or alternatively the seeds are sown in separate rows in the same ratio instead of mix-planting. The corn harvested from the male sterile plants not only have better uniformity than its male fertile counterpart, but also have larger kernel size that is 10-20 mg more than that of its male fertile counterpart. The yield per Mu is increased by 4-10%.

### Industrial applicability

The advantageous benefits of the present invention are:
1. In addition to utilizing the heterosis already displayed by the single-cross hybrid F1 plants, the re-hybridization of the single-cross hybrid gains additional heterosis and meanwhile prevents deterioration of plant vigor due to selfing when the single-cross hybrid is planted alone, thereby the yield also increases by 5-6%.
2. By applying cytoplasmic male sterility in the production of corn, the plant apical dominance is reduced and female ear development is strengthened. Plus the nutrient transfer resulted from the sterile pollen, the potential of kernel yield is increased by about 2-8%.
3. The utilization of pollen-determinant effect of qualitative and quantitative traits in corn production can greatly elevate the content of oil, protein, and other important amino acids. Thus high-oil corn, high-protein corn or enhanced multiple quality trait corn can be produced. The color and appearance of the corn kernel can also be changed. Therefore the nutritional and commercial values of corn are increased.
4. Because the breeders only need to develop the male sterile line and the maintainer line, but not the restorer line for the parents of the single-cross hybrid, the corn breeding program is simplified.
5. By using male sterile hybrids, this production system does not require the labor-intensive detasselling, thereby increases hybrid seed purity.
6. This production system can be applied to any corn hybrid. It is not limited by time and location, nor the new hybrids developed in the future. Any hybrid can produce higher yield and better quality corn by using the method described in the present invention.

## Claims

1. A method of corn production wherein corn seeds of a cytoplasmic male sterile single-cross hybrid are mixed with corn seeds of a male fertile hybrid pollen donor that carries important economic traits in a ratio of 4-9:1 and planted, thereby resulting in corn with high yield and the pollen receiving kernel to exhibit the pollen determinant effects.

2. The method of corn production according to claim 1, wherein the cytoplasmic male sterility includes T-type cytoplasmic male sterility, C-type cytoplasmic male sterility and S-type cytoplasmic male sterility.

3. The method of corn production according to claim 1, wherein said pollen donor corn hybrid seeds refer to seeds of single-cross hybrid, double-cross hybrid, triple-cross hybrid and multi-cross hybrid.

4. The method of corn production according to claim 1, wherein said pollen-determinant effect includes pollen-determinant effects of quantitative traits and pollen-determinant effects of qualitative traits.

5. The method of corn production according to claim 4, wherein said quantitative trait refers to large kernel size, high oil content, high protein content and semi-dominant or cumulative grain quality traits.

6. The method of corn production according to claim 5, wherein said grain quality trait refers to high lysine or high methionine content.

7. The method of corn production according to claim 5, wherein said large kernel size is greater than 350 mg, said high oil content is greater than 6%, said high protein content is greater than 12%.

8. The method of corn production according to claim 6, wherein said high lysine content is greater than 0.26% and said high methionine is greater than 0.20%.

9. The method of corn production according to claim 4, wherein said qualitative traits include major dominant genes controlled maize kernel color aleurone layer traits and the starchy layer traits. Said major dominant genes controlling the color aleurone layer traits are: A₁, A₂, C₁, C₂, B_{z1}, B_{z2}, R₁ and Pr. Said major dominant genes controlling the starchy layer traits are: Y, Wx, O₂, Ae, Sb, Su, and Bt.
